# EUROPEAN PATENT APPLICATION

(11) **EP 2 796 119 A1**
(43) Date of publication of application: **29.10.2014**
(21) Application number: 13164792.7
(22) Date of filing: 22.04.2013
(51) Int. Cl.: A61F 13/00

(54) **Wound dressing and method for manufacturing a wound dressing**

(71) Applicant: Absorbest AB, 590 39 Kisa (SE)
(72) Inventor: Rovaniemi, Rolf, 590 46 Rimforsa (SE)
(74) Representative: WSL Patentanwälte Partnerschaft mbB

(57) **Abstract**

The present invention relates to a wound dressing having an absorbent core with a top surface and a bottom surface wherein the absorbent core comprises a superabsorbent substance, a facing layer, and a backing layer, wherein the facing layer and the backing layer are joint together by a seam, such that the facing layer and the backing layer form a pouch, and wherein the absorbent core is located in the pouch. Due to the seam surrounding the actual absorbent core of the known wound dressings tend to have edges, which do not reach the desired softness of a medical product to be brought into contact with a patient's skin. It is therefore an object of the present invention to provide a wound dressing reducing skin irritation around the wound and a method to manufacture such an improved wound dressing. I order to solve this object it is suggested to further improve a wound dressing of the above kind such that the facing layer is folded covering the entire bottom surface of the absorbent core and comprising at least three sections covering parts of the top surface of the absorbent core, and wherein the seam is at least partly formed between the sections of the facing layer covering parts of the top surface of the absorbent core and the backing layer.

## Description

The present invention relates to a wound dressing having an absorbent core with a top surface and a bottom surface wherein the absorbent core comprises a superabsorbent substance, a facing layer, and a backing layer, wherein the facing layer and the backing layer are joint together by a seam, such that the facing layer and the backing layer form a pouch, and wherein the absorbent core is located in the pouch.

Furthermore, the present invention relates to a method for manufacturing a wound dressing comprising the following steps: providing an absorbent core with a top surface and a bottom surface, wherein the absorbent core comprises a superabsorbent substance, providing a facing layer, providing a backing layer, locating the absorbent core between the facing layer and the backing layer, and joining together the facing layer and the backing layer by a seam such that the facing layer and the backing layer form a pouch, the absorbent core being located in the pouch, such that it is surrounded by the facing layer and the backing layer.

Wound dressings containing superabsorbent polymers in particular for application on heavily secreting wounds have been described in various forms in the prior art. For example the book "More than superabsorbent polymer technology" edited by Frederick L. Buchholz and Andrew T. Graham, published in 1998 by John Wiley & Sons, Inc., ISBN 0-471-19411-5 on page 251 discloses the usage of superabsorbent polymers for the design of effective wound dressings.

WO 03/094813 describes a wound dressing comprising a pouch, in which an absorbent core consisting of a non-woven material comprising superabsorbent polymers dispersed therein is located.

Typically, pouches of the prior art wound dressings containing a superabsorbent substance known from the prior art are formed by forming a seam between the two joining together a facing layer and a backing layer. In order to do so, the facing layer and the backing layer do extend beyond the absorbent core and are joint together, e.g. by using a glue, such that when viewed from the top the glued seam surrounds the area of the absorbent core.

Due to the seam surrounding the actual absorbent core the known wound dressings tend to have edges, which do not reach the desired softness of a medical product to be brought into contact with a patient's skin.

Clinically use of such dressings has shown that due to the hard and stiff boundary area of the wound dressing skin irritation surrounding the actual wound occurs.

It is therefore an object of the present invention to provide a wound dressing reducing skin irritation around the wound and a method to manufacture such an improved wound dressing.

At least one of the above objects is solved by a wound dressing having an absorbent core with a top surface and a bottom surface, wherein the absorbent core comprises a superabsorbent substance, a facing layer and a backing layer, wherein the facing layer and the backing layer are joint together by a seam, such that the facing layer and the backing layer form a pouch, wherein the absorbent core is located in the pouch, wherein the facing layer is folded such that it covers the entire bottom surface of the absorbent core and such that it comprises at least three sections covering parts of the top surface of the absorbent core, and wherein the seam is at least partly formed between the sections of the facing layer covering parts of the top surface of the absorbent core and the backing layer.

The absorbent core may be any structure of material comprising a superabsorbent substance. Superabsorbent substances in the sense of the present application are materials that have the ability to absorb and retain large volumes of water and aqueous solutions. Superabsorbent substances falling into this category are for example modified starch, polymer-like polyvinylalcohol (PVA), polyethylenoxide (PEO) which are all hydrophilic and have a high affinity to water. When chemically or physically cross-linked, these polymers are water-swellable but not water-soluble. The afore-mentioned superabsorbents have been known for a long time.

In a particular embodiment of the present invention the superabsorbent substance is a superabsorbent polymer (SAP) in the form of (granular) particles. In particular such a SAP is made from polymerization of acrylic acids blended with sodium hydroxide in the presence of an initiator to form a poly-acrylic acid sodium salt (sometimes referred to as sodium poly-acrylate).

In an embodiment, the core containing a SAP in the form of particles is formed by at least two non-woven fabric layers or at least two tissue layers or at least two cellulose layers, wherein for fabrication the superabsorbent particles are put on the first layer of a non-woven fabric, then the second layer of the non-woven fabric is put on top and the two layers are consolidated providing a matrix carrying the superabsorbent particles in between the two layers.

A non-woven fabric in the sense of the present application is a material made of at least one layer of long fibres which have been formed to a web and in the next step consolidated. In particular, the consolidation of a non-woven fabric may be achieved by friction and/or cohesion and/or adhesion, for example by needling, felting, spunlacing, melting or pressure melt bonding.

If compared to tissue paper a material will be considered a non-woven fabric in the sense of the present application if more than 50 % of the mass of its fibre components consists of fibres having a ratio of length to their diameter of more than 300. Alternatively, the material will be considered a non-woven fabric in the sense of the present application if this condition is not fulfilled, but if more than 30 % of the mass of its fibres components consists of fibres having a ratio of their length to their diameter of more than 300 and its density is lower than 0.4 g/cm³. This definition corresponds to EN 29 092.

While an absorbent core as described above may be advantageous, it is not excluded to design absorbent cores using different material combinations, as long as the absorbent core contains a superabsorbent substance.

A superabsorbent core extracts and stores any exudates from a wound, to which the wound dressing is applied to. In order to avoid a direct contact between the absorbent core and the wound surface, the wound dressing further comprises a shell or cover enclosing the absorbent core.

According to the present invention, the shell or cover is formed by a facing layer and a backing layer, wherein the facing layer and the backing layer are joint together by a seam, such that the facing layer and the backing layer form a pouch. It is evident, that the absorbent core then is located in the pouch, such that it is surrounded by the facing layer and the backing layer.

It is assumed that in an embodiment the facing layer and/or the backing layer is a laminar structure whose thickness is small compared to its length and width. As such the extension of the facing layer denotes its laminar extension. A direction parallel to the extension of the facing layer and/or the backing layer is essentially parallel to the plane defined by the width and length of the facing layer or backing layer. A direction perpendicular to the extension of the facing layer and/or backing layer essentially denotes the thickness of the facing layer and/or backing layer.

In an embodiment, the facing layer and the backing layer are integrally formed from a single sheet of material. However, in other embodiments the facing layer and the backing layer are two distinct and separate layers, which enables to design their properties and functionalities individually as needed.

In an embodiment, the facing layer is made of a material consisting of one selected of a group comprising a non-woven fabric, a perforated film and a foam based on polyethylene or silicon or a combination thereof.

While it may be that the facing layer is brought into direct contact with the wound, onto which the wound dressing is applied, it may be that the wound dressing comprises one or more further layers between the facing layer and the wound. Depending on its functionality in the wound dressing, a person skilled in the art will choose the material of the facing layer in order to fulfil such functionality.

In an embodiment, the facing layer comprises a non-woven fabric consisting of synthetic and/or cellulose fibres, wherein the fibres of the non-woven fabric are reoriented such that they predominantly extend in a direction perpendicular to the extension of the facing layer. Such a reorientation of the fibres in the non-woven fabric is achieved by orienting the fibres during the fabrication process, in particular during spunlacing or needling.

In an embodiment of the invention, the facing layer comprises a density in a range from 0.1 g/cm³ to 0.6 g/cm³.

In a further embodiment, the facing layer in its unweted state comprises a weight in a range from 12 g/m² to 100 g/m², preferably in a range from 18 g/m² to 70 g/m².

It is further useful if in an embodiment the facing layer comprises a hydrophobic or hydrophilic and/or bacteriocidal or bacteriostatic agent or a combination thereof. This is in particular applicable once the facing layer is brought into direct contact with the wound.

In an embodiment of the present invention, the backing layer serves as a clothing protection. In an embodiment, the backing layer is thus advantageously made of a breathable non-woven fabric or a perforated film enabling a breathing of the wound, but preventing wound exudates from exiting the wound dressing and contaminating a patient's clothing.

While the backing layer may form the outermost layer of the wound dressing, if applicable being brought into contact with the patient's clothing, there may be embodiments, wherein there are further layers on top of the backing layer, i.e. between the backing layer and the patient's clothing, providing additional functionalities for the wound dressing.

The facing layer and the backing layer can be joint together in different ways. If the facing layer and the backing layer consist of an identical material, a single sheet of material can be used, folded and sealed together at its other edges in order to form the pouch. Alternatively two individual sheets, one forming the facing layer, one forming the backing layer, can be connected or joint to each other in order to form the pouch accommodating the absorbent core.

In an embodiment of the invention the absorbent core or the facing layer or any other functional layer in the wound dressing may comprise a substance supporting wound healing, like for example Arnica montana, polyhexanide (PHMB), polyhexamethylen, collagen or chitosan, which are released when the wound dressing is applied to a patient's wound.

In an embodiment of the invention, the seam is formed by gluing or welding the facing layer and the backing layer together.

While in an embodiment the facing layer and/or the backing layer may be in direct contact with the top surface and/or bottom surface of the absorbent core, there may be an embodiment, wherein an additional layer is located between the bottom surface of the absorbent core and the facing layer and/or between the top surface of the absorbent core and the backing layer.

In the prior art, typically the facing layer and the backing layer are joint together by a seam which lies outside the area covered by the absorbent core and thus at least partly encloses or surrounds the absorbent core. In the areas, where the facing layer and the backing layer are connected by the seam, the wound dressing tends to be less flexible than the actual separated layers forming the cover without the seam would be. It has been observed that the area of the seam may lead to a reddening of the patient's skin surrounding the actual wound.

In order to reduce skin irritations by the seam, according to the present invention it is suggested to transfer the seam or the seams such that they predominantly or exclusively are located above the absorbent core when viewed from the wound. According to the present invention, this transfer of the seam is achieved by designing the facing layer, i.e. the layer, which in use of the wound dressing is facing towards the wound, such that it is folded around an edge of the absorbent core. It then covers the entire bottom surface of the absorbent core and comprises at least three sections covering parts of the top surface of the absorbent core. When subsequently the seam or the seams are at least partly formed between the sections of the facing layer covering parts of the top surface of the absorbent core and the backing layer, then the majority of the extension the seam may not get into direct contact with the wound, but is transferred to a position above the absorbent core when viewed from the wound.

While there may be embodiments, wherein some parts of the seam are still formed next to the absorbent core or extending beyond the absorbent core, embodiments may be advisable, wherein the entire seam is exclusively formed between sections of the facing layer covering parts of the top surface of the absorbent core and the backing layer.

In an embodiment, the facing layer has a polygonal shape comprising a plurality of edges, wherein the facing layer is folded along a folding line for each of its edges, each of the folding lines extending essentially in parallel to the corresponding edge.

In a further embodiment, the facing layer has a rectangular shape, wherein the facing layer comprises four sections covering parts of the top surface. Such a rectangular shape of the facing layer with the idea of the present invention may be realized by designing the facing layer, such that its rectangular basic shape along each of its edges comprises flaps, which can be folded, such that they cover part of the top surface of the absorbent core. However, it may be advantageous if in an embodiment the facing layer is rectangular without providing additional flaps along its edges, but the edges of the rectangular facing layer are folded themselves providing overlapping areas between folded sections along adjacent edges. This embodiment provides for a closed and tightly sealed pouch accommodating the absorbent core.

In an embodiment of the invention, the facing layer has an octagonal shape, wherein the facing layer comprises eight sections covering parts of the top surface. As before, these eight sections of the octagonal shape of the facing layer may either be embodied by individual flaps to be folded or the octagon could be folded along its edges, such that a tight sealing along the seams is provided.

The octagonal shape of the facing layer does provide the advantage that when compared for example with a rectangular shape the overlapping areas between adjacent sections of the facing layer being folded on top of the absorbent core are smaller in size, such that the area thickened by the overlapping parts of the sections folded on to the top surface of the absorbent core is reduced. Thus, the octagonal shape of the facing layer provides a more flexible wound dressing than for example a rectangular shape.

In an embodiment of the invention, the backing layer is sized such that it projects beyond at least one of the folding lines of the facing layer. In a particular embodiment it may be preferred, once the backing layer is sized such that it projects beyond all folding lines of the facing layer.

By providing a projection of the backing layer beyond the folding lines of the facing layer, the hard edges of the cover partly formed by the folding lines of the facing layer are protected by the areas of the backing layer extending beyond the folding lines.

In an embodiment, the backing layer has an essentially rectangular shape comprising four edges. In an embodiment, such a backing layer may comprise rounded corners. Such an embodiment of the backing layer is in particular useful when combined with a rectangular-shaped facing layer as it provides a soft and appealing product.

When absorbing liquid, the absorbent core will extend its dimensions predominantly in a direction perpendicular to its lateral extension, i.e. it will get thicker. In order to accommodate for such a swelling of the absorbent core when absorbing liquid, in particular exudates from a wound, in an embodiment of the wound dressing, the backing layer is folded along four folding lines spanning from one edge of the backing layer to another edge of the backing layer, wherein each two of the folding lines provide for a zig-zag folding of the backing layer, and wherein two folding lines located the furthest apart from each other before folding of the top layer are located closest to each other after folding of the top layer.

This way a volume of the pouch is formed, which is extendable, in particular extendable by a swelling or thickening of the absorbent core.

While this extendable backing layer is advantageous an embodiment of a wound dressing as described before, wherein at least part of the seam between the facing layer and the backing layer is located above the absorbent core when viewed in a direction from the wound, the expendable backing layer could also be embodied with a wound dressing comprising a seam which is essentially located in the same plane as the absorbent core.

In a further embodiment of the invention, the backing layer is folded along two pairs of folding lines, wherein the folding lines of the two pairs do form an angle therebetween, preferably an angle of about 90°.

At least one of the above objects is also solved by a method for manufacturing a wound dressing comprising the following steps: providing an absorbent core with a top surface and a bottom surface, wherein the absorbent core comprises a superabsorbent substance, providing a facing layer, providing a backing layer, locating the absorbent core between the facing layer and the backing layer, and joining together the facing layer and the backing layer by a seam such that the facing layer and the backing layer form a pouch, the absorbent core being located in the pouch, wherein before joining the facing layer and the backing layer together the facing layer is folded such that it covers the entire bottom surface of the absorbent core and such that it comprises at least three sections covering parts of the top surface of the absorbent core and wherein the seam is at least partly formed between the sections of the facing layer covering parts of the top surface of the absorbent core and the backing layer.

As far as aspects of the present invention have been described above regarding the wound dressing, they also apply to a process for manufacturing such wound dressing and vice versa.

In an embodiment of the inventive method, before joining the facing layer and the backing layer together, the backing layer is folded along four folding lines spanning from one edge of the backing layer to another edge of the backing layer, wherein each two of the folding lines provide for a zig-zag folding of the backing layer, and wherein two folding lines located furthest apart from each other before folding of the backing layer are located closest to each other after folding of the backing layer. The term furthest and closest with respect to this embodiment are considered in a direction essentially parallel to the top surface of the absorbent core.

In a further embodiment, the backing layer is folded along two pairs of folding lines, wherein the folding lines of the two pairs do form an angle between each other, preferably an angle of about 90°.

This way, a backing layer is provided, which allows to expand in two distinct, preferably orthogonal, directions.

Further advantages, features and applications of the present invention will become apparent from the following description of embodiments and the corresponding figures attached.
Figures 1 to 7 schematically show individual steps for manufacturing of a wound dressing according to a first embodiment of the present invention.
Figure 8 shows a schematic view of a wound dressing according to a second embodiment of the present invention.
Figures 9 to 11 schematically show individual steps for manufacturing a further wound dressing according to an embodiment of the present invention.
Figures 12 and 13 schematically show steps for manufacturing a wound dressing according to a further embodiment of the present invention having an expandable backing layer.
Figures 14 to 16 schematically show steps for manufacturing a wound dressing according to yet another embodiment of the present invention having an expandable backing layer.
Figures 17 to 20 schematically show steps for manufacturing a wound dressing according to a further embodiment of the present invention comprising two facing layers and two absorbent cores.

In the figures, identical elements are denoted by identical reference numbers.

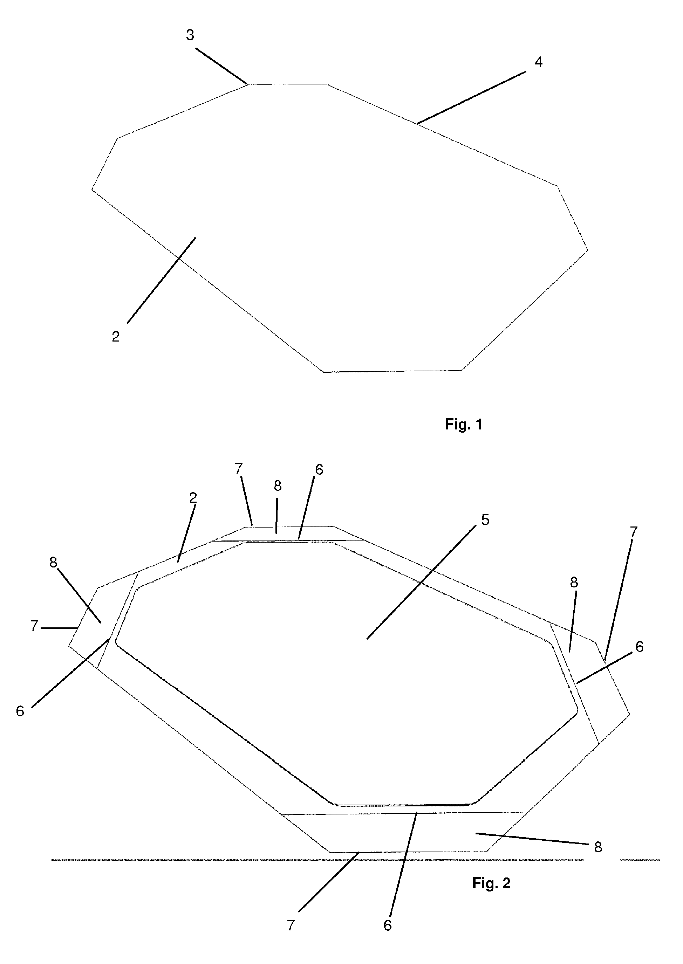

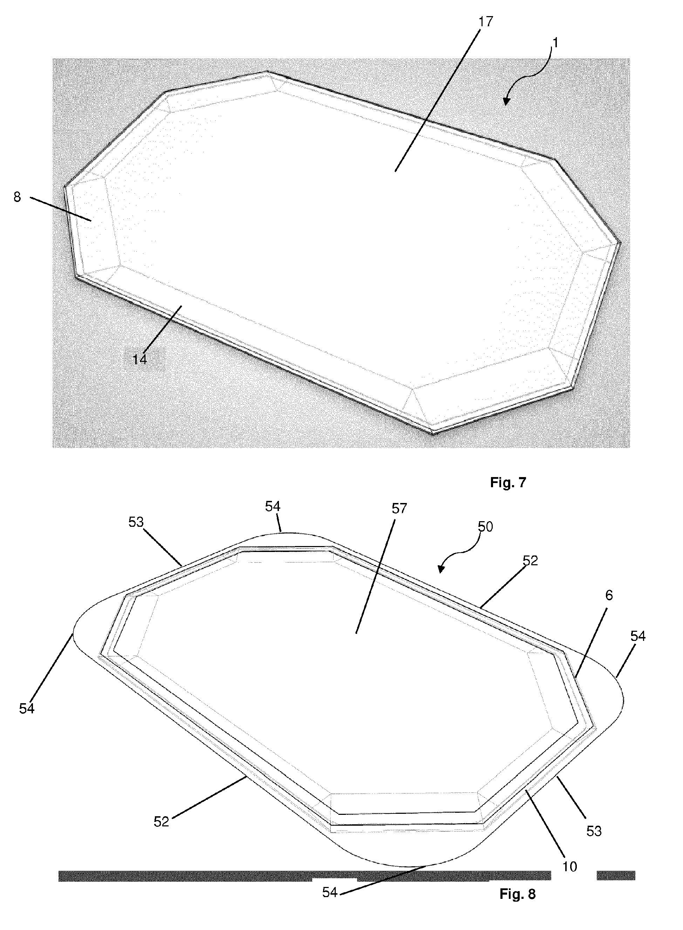

Figures 1 to 6 show different steps necessary to produce a wound dressing 1 according to a first embodiment of the present invention. While in the different figures the different steps for manufacturing the wound dressing 1 are shown, the completed wound dressing 1 is schematically depicted in figure 7.

Figure 1 shows the facing layer 2 of the wound dressing being provided at the beginning of the manufacturing process. The facing layer 2 in this specific example is a sheet of a non-woven being permeable for any exudates exudating from a patient's wound. When applying the wound dressing 1 to a patient's wound, the facing layer 2 is facing towards the actual wound surface and is brought in contact with the wound surface.

The facing layer 2 of the first embodiment has an octagonal shape comprising eight corners 3 as well as eight edges 4.

In a second manufacturing step as depicted in figure 2, the actual absorbent core 5 is laid on top of the facing layer 2. While in principle the absorbent core 5 may be fixed at the facing layer 2, e.g. by using one or a plurality of dots of glue, in the embodiment as depicted in figures 1 to 7 the absorbent core 5 is loosely laid onto the facing layer 2.

In this particular embodiment, the absorbent core 5 contains a SAP in the form of particles. It is formed by two non-woven fabric layers, wherein for fabrication the superabsorbent particles are dispersed on top of the first layer of a non-woven fabric. Then the second layer of the non-woven fabric is put on top and the two layers are consolidated providing a matrix carrying the superabsorbent particles in between the two layers. As an alternative for a non-woven fabric manufacturing of the absorbent core could be performed by using at least two sheets or layers of cellulose or tissue (paper).

As visible in figure 2, the facing layer 2 has a first set of four folding lines 6. Each of the folding lines 6 in the first set of folding lines does extend essentially parallel to the short edges 7 of the facing layer 2. In the present example, the folding lines 6 are folds produced by a corresponding mechanical tool.

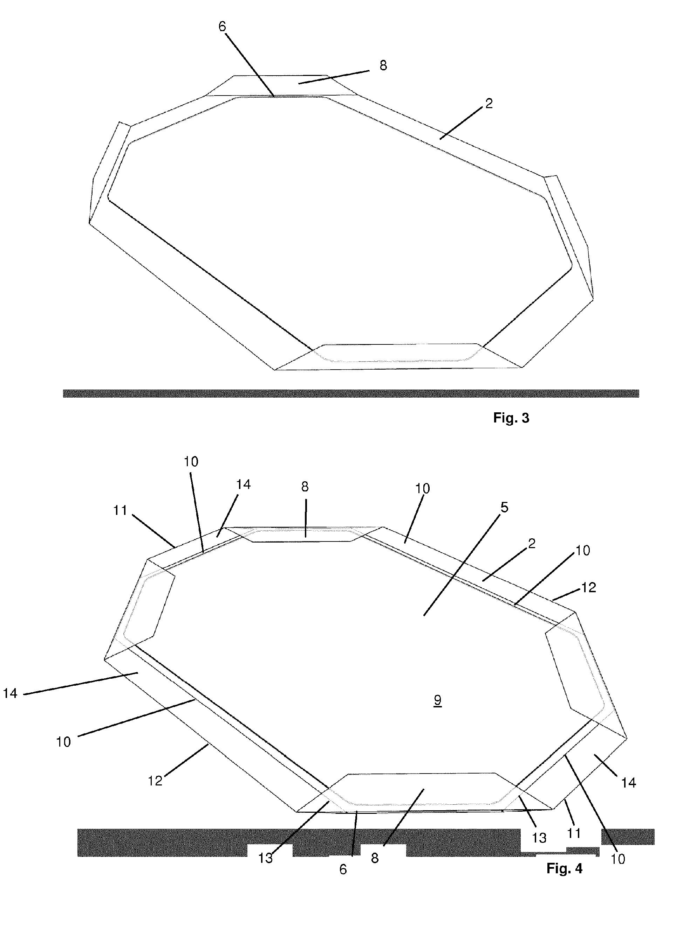

By the folding lines sections 8 of the facing layer 2 are formed, which can be folded, such that they pivot around the folding lines 6 as depicted in figure 3. These sections 8 of the facing layer 2 finally do cover parts of the top surface 9 of the absorbent core 5 as shown in figure 4.

This way, the facing layer 2 covers the entire bottom surface (not visible in the figures) of the absorbent core 5 and parts of the top surface 9 of the absorbent core 5.

As shown in figure 4, the facing layer 2 further comprises a second set of folding lines 10 extending essentially parallel to the remaining four edges 11, 12 of the facing layer 2.

While the folding lines 10 of the second set of folding lines have been depicted for the first time in figure 4, it is evident for a person skilled in the art that in a real world manufacturing process the folding lines 6, 10 of the first and second sets may be formed, e.g. by embossing, in the facing layer right in the beginning of the process either before or after cutting the facing layer 2 to its desired size and shape.

However, alternatively, the second set of folding lines 10 may be formed on the facing layer at a stage as depicted in figure 4. Forming the folding lines 10 at this stage may be advantageous as it would not only provide the folding lines 10, but also additional folding lines 13 along those sections 8 of the facing layer, which have already been pivoted around the folding lines 6 and folded on top of the absorbent core 5. By the second set of folding lines 10 four further sections 14 of the facing layer are formed, which are pivotable around the folding lines 10.

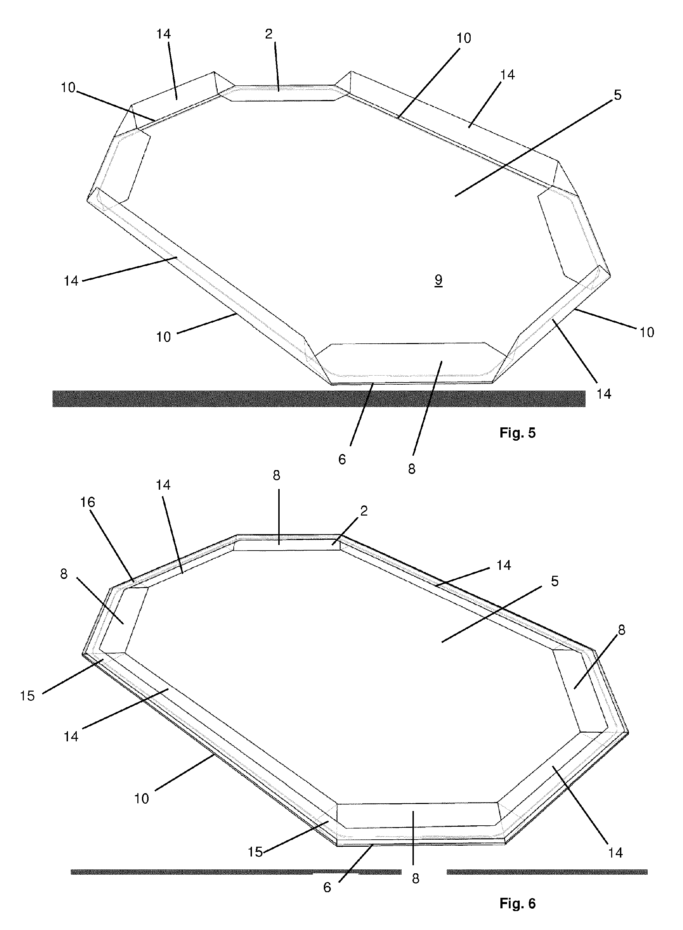

In the next step as shown in figure 5, these additional four sections 14 are pivoted around the folding lines 10 such that they are folded on top of the absorbent core 5 covering parts of the top surface 9 of the absorbent core 5.

Figure 6 shows the result of folding all sections 8, 14, such that they are covering parts of the top surface 9 of the absorbent core 5. When considered in this position the folding lines 6, 10 of the facing layer 2 do form the edges of a - still open - pouch accommodating the absorbent core 5.

From figure 6 it is apparent that the octagonal shape being representative for a generally polygonal shape reduces the area, wherein adjacent folded sections 8, 14 do overlap and thus thicken the wound dressing. These overlapping sections are denoted by reference number 15 in figure 6. Figure 6 further shows that the facing layer 2 after the above manufacturing steps covers an area which has approximately the same shape as the absorbent core 5.

In order to close the pouch formed at the stage represented by figure 6, a glue 16 is applied onto the sections 8, 14 covering parts of the top surface 9 of the absorbent core 5 in order to provide adhesion of the facing layer 2 to a backing layer 16 (not depicted in figure 6). Figure 7 shows a schematic view of the completed wound dressing 1, wherein a backing layer 16 has been applied and glued by the glue line 16 forming a seam onto the facing layer 2.

In figure 7, the backing layer 17 is depicted semi-transparent such that sections 8 and 14 of the facing layer 2 to which the backing layer 17 is glued are visible.

It is apparent from figure 7, that the wound dressing 1 according to this embodiment only comprises a seam or a glue line 16 which when viewed from a direction from the facing layer 2 and thus from the wound exclusively lies above the plane of the absorbent core 5. Thus no seam or glue line 16 is brought into contact with a patient's skin when the wound dressing 1 is applied to a wound with the facing layer 2 being in contact with the wound.

Figure 8 shows another embodiment of the wound dressing 50, wherein the facing layer 2 and the absorbent core 5 are identical to the embodiment as depicted in figures 1 to 7, but a backing layer 57 being different in shape has been attached in order to complete the pouch accommodating the absorbent core 5.

Thus figures 1 to 6 do also represent the individual steps of manufacturing a wound dressing 50 according to figure 8, while in the last step a different backing layer 57 is applied. The backing layer 57 has a generally rectangular shape providing four edges 52, 53 and rounded corners 54. The area covered by the backing layer 57 is slightly larger than the area covered by the folded facing layer 2. Thus, the backing layer 57 when glued on top of the facing layer 2 projects beyond the folding lines 6, 10 of the facing layer which after folding of the facing layer 2 do form the edges surrounding the absorbent core 5.

By providing these sections 52 of the backing layer 57 extending or projecting beyond the folding lines 6, 10 of the facing layer, a wound dressing is provided, having flexible edges 52, 53, such that the product provides an appealing and soft handling. The shape of this embodiment enhances a patient's comfort when wearing a wound dressing.

For all embodiments depicted with reference to the figures, the backing layer operates as a clothing protection. It thus is made of a backing material being breathable , but at the same time preventing wound exudates from exiting the wound dressing and contaminating a patient's clothing.

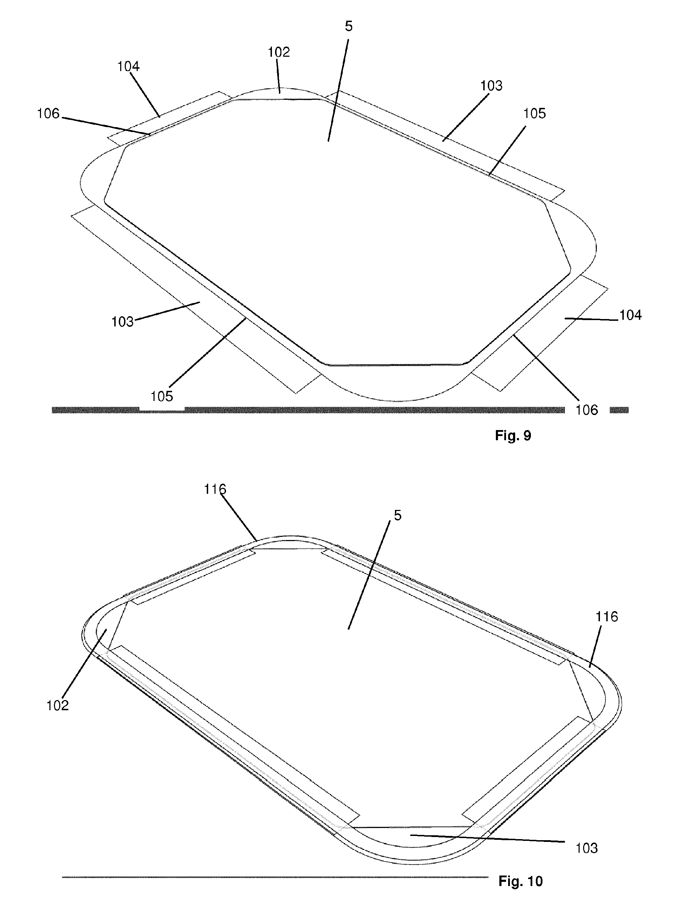

Figures 9 to 11 show a third embodiment of a wound dressing 100 according to the present invention. In this embodiment, the facing layer 102 as visible from figure 9 for example has a general rectangular shape being provided with additional flaps 103, 104, which can be pivoted around folding lines 105, 106 in order to be folded on top of the absorbent core 5 each covering a part of the top surface 9 of the absorbent core 5.

Figure 10 shows the flaps 104, 103 in the folded condition. It is apparent from figure 10, that a seam now applied onto the facing layer will mostly be applied onto the sections of the facing layer 102 covering parts of the absorbent core 5, but some remaining bits of the glue line or seam 116 are applied onto section 103 of the facing layer 102 not being folded. Thus, in these sections 103 of the facing layer 102 the glue line is not formed above the absorbent core 5 but in the same plane as the absorbent core.

While still covered by the general concept of the present invention, this embodiment of a wound dressing 100 has tiny sections, wherein parts of the facing layer 102 covered by the glue line 116 are in an indirect contact with a patient's skin when the wound dressing 100 is applied to a wound.

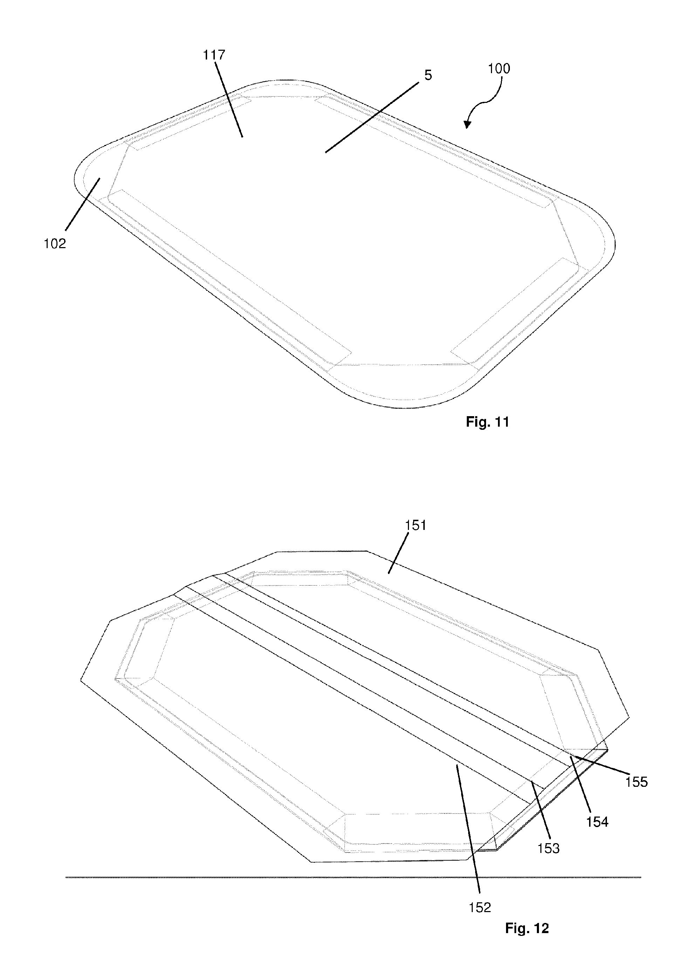

Figure 11 schematically shows the completed wound dressing 100 having a backing layer 117 being mounted on top of the facing layer 102. In figure 11 again the backing layer 117 is shown being semi-transparent, such that the further components 5, 102 of the wound dressing 100 are visible. The backing layer 107 essentially has an area being identical to the area of the facing layer 102, when the flaps 103, 104 are folded on top of the absorbent core 5.

In the embodiment according to figures 9 to 11 the absorbent core 5 is identical to the absorbent core 5 of the embodiments discussed above.

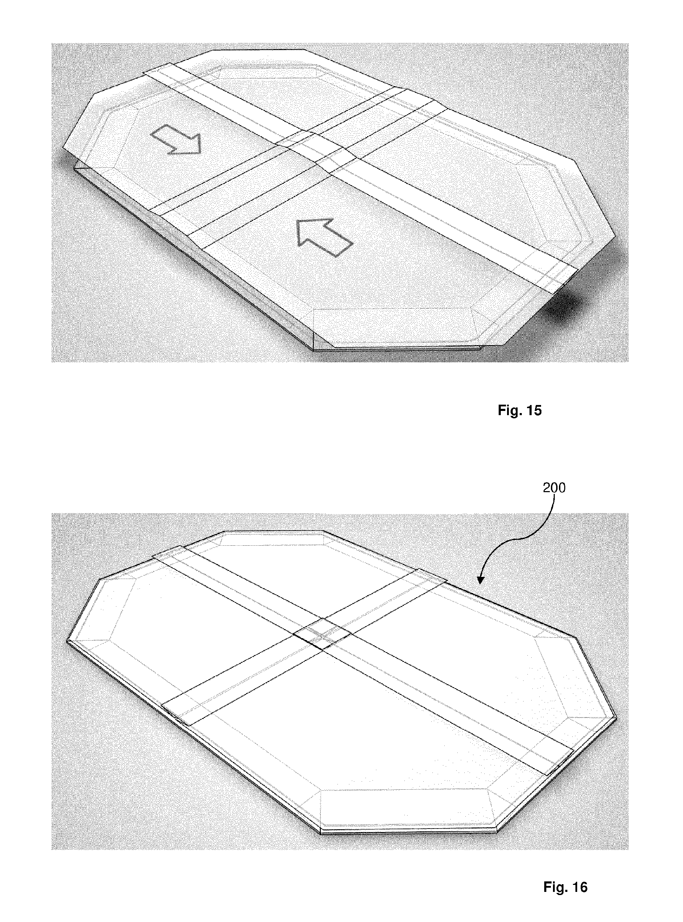

Figures 12 and 13 and 14 to 16, respectively show two further embodiments of wound dressings 150, 200 being based on a combination of a facing layer 2 and an absorbent core 5 as depicted for the embodiments of figures 1 to 7, and 8, respectively. The two wound dressings 150, 200 do differ from the wound dressing 1 of figures 1 to 7 by the forming of their respective backing layers 150,201.

When absorbing any liquid, in particular exudates from a wound, the absorbent core 5 of a wound dressing according to the examples depicted will swell predominantly in a direction perpendicular to the lateral extension of the dressing. When swelling the absorbent core 5 will start filling the volume of the pouch formed by the facing layer and the backing layer.

In order to provide a volume of the pouch formed, which is extendable during swelling of the absorbent core 5, both backing layers 151, 201 according to the embodiments of figures 12 to 16 do comprise a zig-zag-folding, which is described in more detail below.

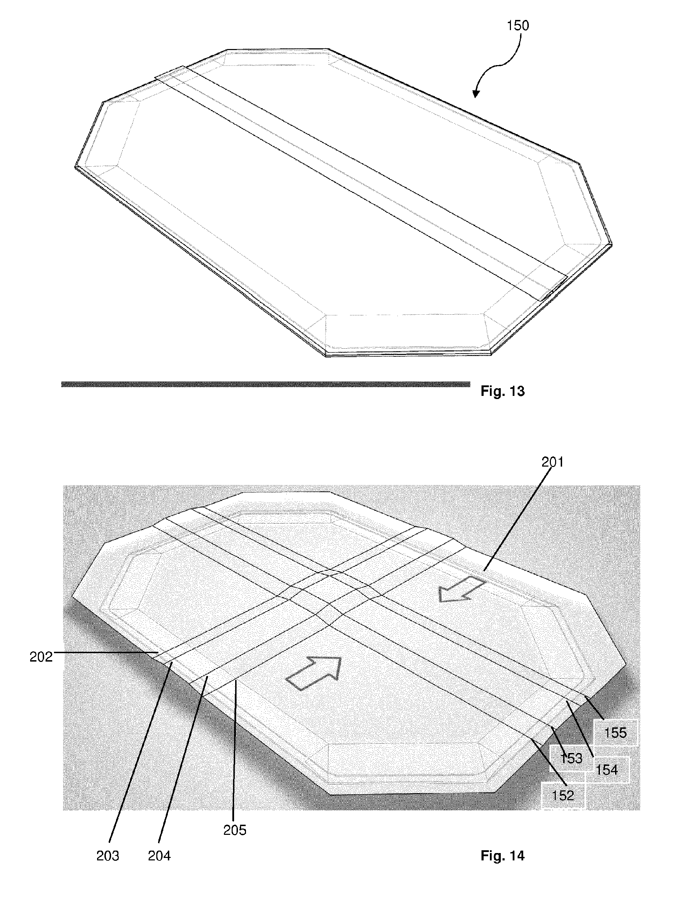

The backing layer of the embodiment of a wound dressing 150 depicted in figures 12 and 13 comprises four essentially parallel folding lines 152 to 155. Of these four folding lines the two folding lines 152, 155 and the two folding lines 153, 154 do form a pair in that the pivoting directions of the sections of the backing layer 151 adjacent to the lines have opposite pivoting directions.

While the backing layer 151 is folded about the folding lines 152 to 155, such that a section of the backing layer is folded onto the top surface of the backing layer another part of the backing layer 151 adjacent to the folding lines 153, 154, is folded, such that it is located on top of the inner surface of the backing layer 151. Explained differently the two outermost folding lines 152, 155 before folding will be located closest together after folding of the backing layer 151.

This way two zig-zag-foldings are applied to the backing layer 151 which enables an expansion of the volume of the pouch formed by the backing layer 151 and the facing layer 2. When swelling the absorbent core 5 will simply push the backing layer 151 outwardly, such that the double zig-zag-folding expands and provides an extended volume of the pouch.

While the wound dressing 150 according to figures 12 and 13 does provide the backing layer 151 being expandable in one direction, the backing layer 201 of the embodiment according to figures 14 to 16 has two essentially orthogonal sets of zig-zag-foldings. Thus it provides for a backing layer 201 being extendable in two directions.

In order to do so, the backing layer 201 of the wound dressing 200 comprises two sets of four folding lines each. The first set of folding lines being identical to the folding lines of the backing layer 151 of the embodiment according to figures 12 and 13 comprises individual lines denoted by reference number 152 to 155 while the lines of the second set are denoted by reference numbers 202 to 205. As can be seen from the figures, the folding lines 152, 155 and 202 to 205, respectively, do span across the backing layers 151, 201 from one edge to another.

When gluing the backing layers 151, 201 onto the facing layers 2, care has to be taken to provide an entirely closed or sealed pouch by carefully gluing the zig-zag-areas along the edges of the dressings 150, 200.

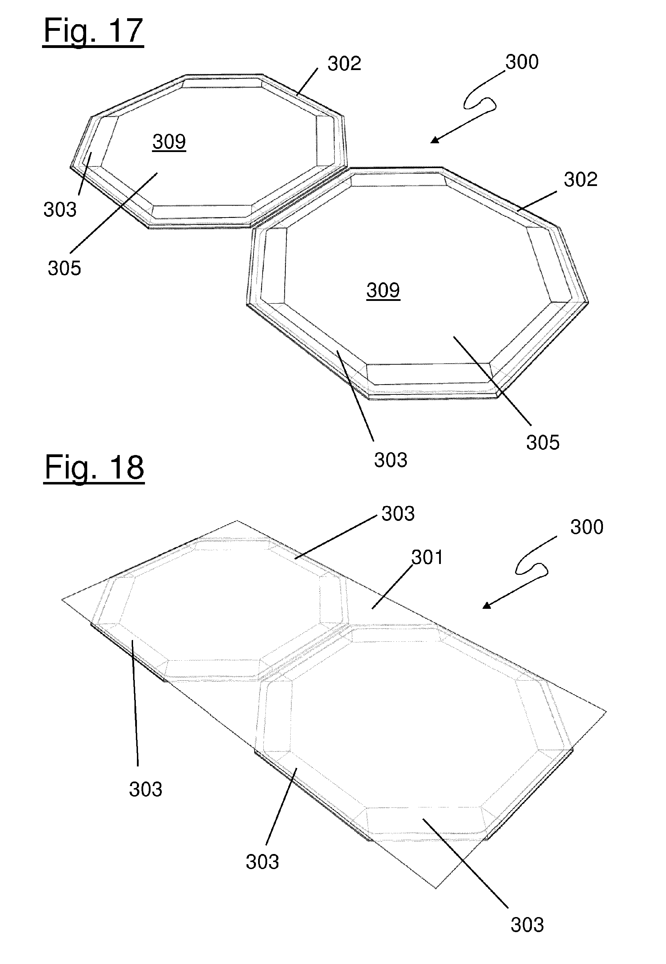

Figures 17 to 20 show yet another embodiment of a wound dressing 300 according to an embodiment of the present invention.

In figure 17 two readily folded facing layers 302 are shown, wherein each of the facing layers 302 accommodates an individual absorbent core 305. The two combinations of a facing layer 302 and an absorbent core 305 each are depicted in a manufacturing step as for example depicted for another embodiment in figure 6. So in principle all manufacturing steps which have been described above with respect to figures 1 to 5 have been performed before in order to provide the two combinations of a facing layer 302 and an absorbent core 305 as depicted in figure 17.

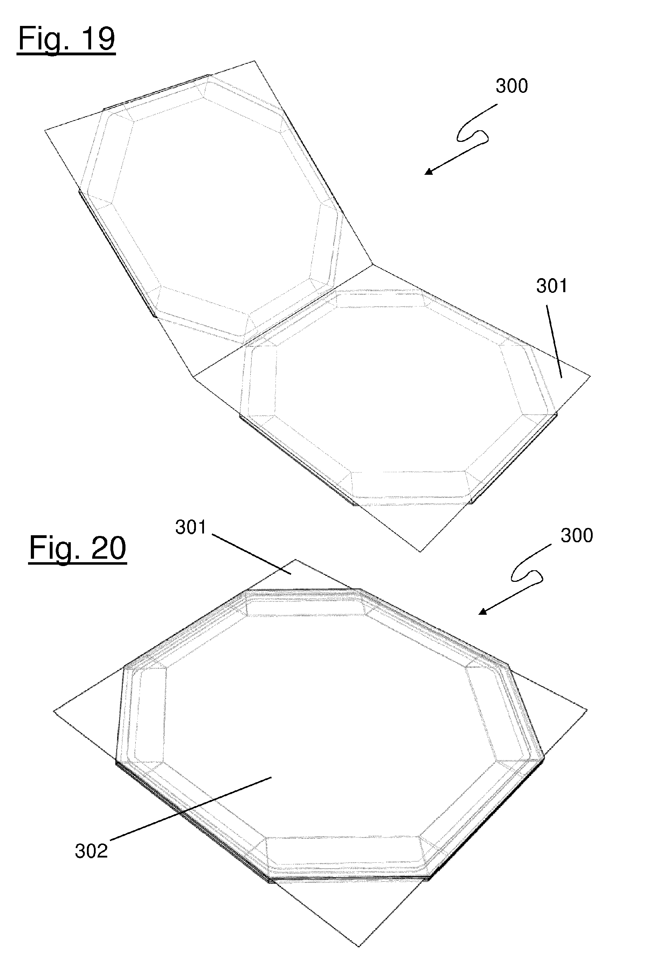

In figure 18 the dressing 300 is shown as being completed by gluing a backing layer 301 on top of those folded sections 303 of the facing layers, which are folded onto of the top surfaces 309 of the absorbent cores 305. Via the backing layer 301 the two facing layers 302 are connected to form a single wound dressing. The two halves of the dressing each comprise an absorbent core 305, a facing layer 302. A single continuous backing layer 301 can be folded as depicted in figure 19 in order to end up with a completed wound dressing 300 as depicted in figure 20. The two halves of the backing layer 301 are glued onto each other, such that the resulting wound dressing 300 does have two facing layers 302 pointing into opposite directions. Thus, the wound dressing may be applied on either of its two sides onto a wound.

For purposes of original disclosure it is pointed out that all features which are apparent for a person skilled in the art from the present description, the figures and the claims, even if they have only been described with further features, could be combined on their own or together with all the combinations of the features disclosed herein, if not excluded explicitly or technically impossible. A comprehensive explicit description of all possible combinations or features is only omitted in order to provide readability of the description.

**List of reference numbers**

| | |
|---|---|
| 1, 50, 100, 150, 200, 300 | wound dressing |
| 2, 102, 302 | facing layer |
| 3, 54 | corners |
| 4, 11, 12, 52, 53 | edges |
| 5, 305 | absorbent core |
| 6, 10, 13, 105, 106, 152 153, 154, 155, 202, 203, 204, 205 | folding lines |
| 7 | short edges |
| 8, 14, 303 | folded sections of the facing layer 2 |
| 9 | top surface of the absorbent core 5 |
| 15 | overlapping sections of folded sections 8, 14 |
| 16, 116 | glue line or seam |
| 17, 57, 107, 117, 201, 151, 301 | backing layer |
| 103, 104 | foldable flaps |

## Claims

1. A wound dressing (1, 50, 100, 150, 200, 300) having
an absorbent core (5) with a top surface (9) and a bottom surface, wherein the absorbent core comprises a superabsorbent substance,
a facing layer (2, 102), and
a backing layer (16, 51, 107, 117, 201, 151)
wherein the facing layer (2, 102) and the backing layer (16, 51, 107, 117, 201, 151)are joint together by a seam such that the facing layer (2, 102) and the backing layer (16, 51, 107, 117, 201, 151) form a pouch, and
wherein the absorbent core (5) is located in the pouch,
**characterised in that**
the facing layer (2, 102) is folded such that it covers the entire bottom surface of the absorbent core (5) and such that it comprises at least three sections (8, 14) covering parts of the top surface (9) of the absorbent core (5),
wherein the seam is at least partly formed between the sections (8, 14) of the facing layer (2, 102) covering parts of the top surface (9) of the absorbent core (5) and the backing layer (16, 51, 107, 117, 201, 151).

2. A wound dressing (1, 50, 100, 150, 200, 300) according to claim 1, wherein the facing layer (2, 102) has a polygonal shape comprising a plurality of edges (4, 11, 12, 52, 53) and wherein the facing layer (2, 102) is folded along a folding line (204, 205) for each of its edges (4, 11, 12, 52, 53), each of the folding lines (204, 205) extending essentially in parallel to the corresponding edge (4, 11, 12, 52, 53).

3. A wound dressing (1, 50, 100, 150, 200, 300) according to claim 1 or 2, wherein the facing layer (2, 102) has a rectangular shape and wherein the facing layer (2, 102) comprises four sections (8, 14) covering parts of the top surface (9).

4. A wound dressing (1, 50, 100, 150, 200, 300) according to claim 1 or 2, wherein the facing layer (2, 102) has an octagonal shape and wherein the facing layer (2, 102) comprises eight sections (8, 14) covering parts of the top surface (9).

5. A wound dressing (1, 50, 100, 150, 200, 300) according to one of the previous claims, wherein the seam is exclusively formed between sections (8, 14) of the facing layer (2, 102) covering parts of the top surface (9) of the absorbent core (5) and the backing layer (16, 51, 107, 117, 201, 151).

6. A wound dressing (1, 50, 100, 150, 200, 300) according to one of the previous claims, wherein the backing layer (16, 51, 107, 117, 201, 151) is sized such it projects beyond the folding lines (204, 205) of the facing layer (2, 102).

7. A wound dressing (1, 50, 100, 150,200, 300) according to one of the previous claims, wherein the backing layer (16, 51, 107, 117, 201, 151) has an essentially rectangular shape comprising four edges.

8. A wound dressing (1, 50, 100, 150 ,200) according to claim 7, wherein the backing layer (16, 51, 107, 117, 201, 151) comprises rounded corners.

9. A wound dressing (1, 50, 100, 150, 200, 300) according to one of the previous claims, wherein the backing layer (16, 51, 107, 117, 201, 151) is folded along four folding lines (204, 205) spanning from one edge (4, 11, 12, 52, 53) of the backing layer (16, 51, 107, 117, 201, 151) to another edge (4, 11, 12, 52, 53) of the backing layer (16, 51, 107, 117, 201, 151), wherein each two of the folding lines (204, 205) provide for a zig-zag folding of the backing layer (16, 51, 107, 117, 201, 151) and wherein two folding lines (204, 205) located furthest apart from each other before folding of the top layer are located closest to each other after folding of the top layer.

10. A wound dressing (1, 50, 100, 150, 200, 300) according to claim 9, wherein the backing layer (16, 51, 107, 117, 201, 151) is folded along two pairs of four folding lines (204, 205), wherein the folding lines (204, 205) of the two pairs do form an angle there between, preferably an angle of about 90°.

11. A wound dressing (1, 50, 100, 150, 200, 300) according to one of the previous claims, wherein the seam is formed by gluing or welding the facing layer (2, 102) and the backing layer (16, 51, 107, 117, 201, 151) together.

12. A method for manufacturing a wound dressing (1, 50, 100, 150, 200, 300) comprising the following steps:
providing an absorbent core (5) with a top surface (9) and a bottom surface, wherein the absorbent core comprises a superabsorbent substance,
providing a facing layer (2, 102),
providing a backing layer (16, 51, 107, 117, 201, 151),
locating the absorbent core (5) between the facing layer (2, 102) and the backing layer (16, 51, 107, 117, 201, 151), and
joining together the facing layer (2, 102) and the backing layer (16, 51, 107, 117, 201, 151) by a seam such that the facing layer (2, 102) and the backing layer (16, 51, 107, 117, 201,151) form a pouch, the absorbent core (5) being located in the pouch, **characterised in that**
before joining the facing layer (2, 102) and the backing layer (16, 51, 107, 117, 201, 151) together the facing layer (2, 102) is folded such that it covers the entire bottom surface of the absorbent core (5) and such that it comprises at least three sections (8, 14) covering parts of the top surface (9) of the absorbent core (5) and
wherein the seam is at least partly formed between sections (8, 14) of the facing layer (2, 102) covering parts of the top surface (9) of the absorbent core (5) and the backing layer (16, 51, 107, 117, 201, 151).

13. Method according to claim 12, wherein before joining the facing layer (2, 102) and the backing layer (16, 51, 107, 117,201, 151) together the backing layer (16, 51, 107, 117, 201, 151) is folded along four folding lines (204, 205) spanning from one edge (4, 11, 12, 52, 53) of the backing layer (16, 51, 107, 117, 201, 151) to another edge (4, 11, 12, 52, 53) of the backing layer (16, 51, 107, 117, 201, 151) wherein each two of the folding lines (204, 205) provide for a zig-zag folding of the backing layer (16, 51, 107, 117, 201, 151), and wherein two folding lines (204, 205) located furthest apart from each other before folding of the top layer are located closest to each other after folding of the top layer.

14. Method according to claim 12 or 13, wherein the backing layer (16, 51, 107, 117, 201, 151) is folded along two pairs of four folding lines (204, 205), wherein the folding lines (204, 205) of the two pairs do form an angle there between, preferably an angle of about 90°.
